# EUROPEAN PATENT APPLICATION

(11) **EP 4 451 205 A1**
(43) Date of publication of application: **23.10.2024**
(21) Application number: 24170286.9
(22) Date of filing: 15.04.2024
(51) Int. Cl.: G06T 3/06, G06T 5/90, G06T 7/10

(54) **MEDICAL IMAGE TRANSLATION METHOD AND APPARATUS**

(30) Priority: 21.04.2023 KR 20230052818
(71) Applicant: Medicalip Co., Ltd., Gangwon-do 24341 (KR)
(72) Inventor: PARK, Sang Joon, Seoul (KR); KIM, Jong Min, Yongin-si, Gyeonggi-do (KR); WITANTO, Joseph Nathanael, Seoul (KR)
(74) Representative: Patentanwälte Ruff, Wilhelm, Beier, Dauster & Partner mbB

(57) **Abstract**

Provided are a medical image translation method and apparatus. The medical image translation apparatus (100) receives a first medical image (120) and translates the first medical image into a second medical image (130) through an image translation model (110). The first medical image is a two-dimensional (2D) medical image, and the second medical image is a 2D medical image obtained by reconstructing a brightness value of each of pixels of the first medical image while maintaining a structure shown in the first medical image. The image translation model is a model implemented with an artificial neural network that reflects and outputs a feature of a brightness value of each of pixels of a reference image trained in a training process in a 2D medical image.

## Description

The invention relates to a medical image translation method and apparatus, and more particularly, to a method and apparatus for translating a medical image to be suitable for an artificial intelligence model. This application is based on and claims priority to Korean Patent Application No. 10-2023-0052818, filed on April 21, 2023, in the Korean Intellectual Property Office, the disclosure of which is incorporated by reference herein in its entirety.

Artificial intelligence models implemented with artificial neural networks are used in the medical field to segment human tissues or organs or to diagnose or detect lesions. In order to use artificial intelligence models in the medical field, a process of training artificial intelligence models by using training data should be preceded. Training data used to train artificial intelligence models may have different textures such as brightness values. For example, a medical image captured by first image capturing equipment (e.g., a computed tomography (CT) device or a magnetic resonance imaging (MRI) device) may be used to train a first artificial intelligence model, and a medical image captured by second image capturing equipment (e.g., an X-ray device) may be used to train a second artificial intelligence model. According to a type of image capturing equipment, there may be a difference in brightness or clarity between images even when the images are images of the same body part. Accordingly, an optimal result may be obtained when inputting a medical image captured by the first image capturing equipment, rather than a medical image captured by the second image capturing equipment, to the first artificial intelligence model that has been trained by using medical images captured by the first image capturing equipment. However, it is practically impossible to create training data for each image capturing equipment and train an artificial intelligence model for each image capturing equipment.

It is the technical problem underlying the invention to provide a method and apparatus for translating a texture such as a brightness while maintaining a structure of a medical image to be suitable for an artificial intelligence model.

The invention solves this problem by providing a medical image translation method having the features of claim 1 and a medical image translation apparatus having the features of claim 8. Additional aspects will be set forth in part in the description which follows and, in part, will be apparent from the description, or may be learned by practice of the presented embodiments. Advantageous embodiments of the invention are mentioned in the dependent claims the wording of which is herewith incorporated into the description to avoid unnecessary text repetition.

According to an aspect of the invention, a medical image translation method includes receiving a first medical image, and obtaining a second medical image by translating the first medical image through an image translation model, wherein the first medical image is a two-dimensional (2D) medical image, and the second medical image is a 2D medical image obtained by reconstructing a brightness value of each of pixels of the first medical image while maintaining a structure of the first medical image, wherein the image translation model is a model implemented with an artificial intelligence network that reflects and outputs a feature of a brightness value of each of pixels of a reference image trained in a training process in a 2D medical image.

According to another aspect of the invention, a medical image translation apparatus includes an input unit configured to receive a first medical image, and an image translation model configured to translate the first medical image into a second medical image, wherein the first medical image is a two-dimensional (2D) medical image, and the second medical image is a 2D medical image obtained by translating a brightness value of each of pixels of the first medical image while maintaining a structure of the first medical image, wherein the image translation model is a model implemented with an artificial intelligence network that reflects and outputs a feature of a brightness value of each of pixels of a reference image used in a training process in a 2D medical image.

The above and other aspects, features, and advantages of the invention will be more apparent from the following description of exemplary embodiments explained in conjunction with the accompanying drawings, in which:
FIG. 1 is a diagram illustrating an example of a medical image translation apparatus, according to an embodiment;
FIG. 2 is a diagram illustrating an example of an image translation model, according to an embodiment;
FIG. 3 is a diagram illustrating an example of a method of training an image translation model, according to an embodiment;
FIG. 4 is a diagram illustrating another example of a method of training an image translation model, according to an embodiment;
FIG. 5 is a diagram illustrating an example of a segmentation model, according to an embodiment;
FIG. 6 is a diagram illustrating an example of a method of generating a medical image suitable for a segmentation model by using an image translation model, according to an embodiment;
FIG. 7 is a diagram illustrating a configuration of an example of a medical image translation apparatus, according to an embodiment;
FIG. 8 is a view illustrating an example of a translation result of an image translation model, according to an embodiment; and
FIGS. 9 and 10 are views illustrating an example of a segmentation result of a segmentation model, according to an embodiment.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

A medical image translation method and apparatus according to an embodiment will now be described in detail with reference the accompanying drawings.

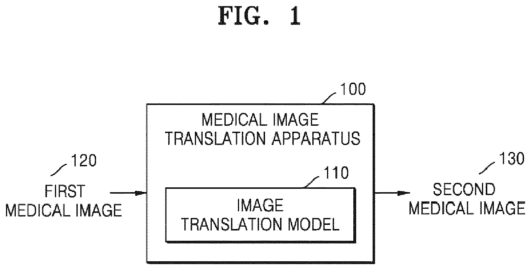

FIG. 1 is a diagram illustrating an example of a medical image translation apparatus, according to an embodiment.

Referring to FIG. 1, a medical image translation apparatus 100 translates a first medical image 120 into a second medical image 130 by using an image translation model 110. The first medical image 120 may be a two-dimensional (2D) or three-dimensional (3D) medical image. Examples of the 2D medical image may include an x-ray image. Examples of the 3D medical image may include a computed tomography (CT) image, an magnetic resonance imaging (MRI) image, and an ultrasound image. Various other types of 2D or 3D medical images may exist and are not limited to specific examples. However, for convenience of explanation, the following will be described assuming that the first medical image 120 is an x-ray image.

The medical image translation apparatus 100 may receive the first medical image 120 from a picture archiving and communication system (PACS) or from an external server or an external terminal through a wired/wireless communication network. Alternatively, the medical image translation apparatus 100 may receive the first medical image 120 from an external storage medium through a universal serial bus (USB) port or the like. Various other methods of receiving the first medical image 120 may be applied to the present embodiment.

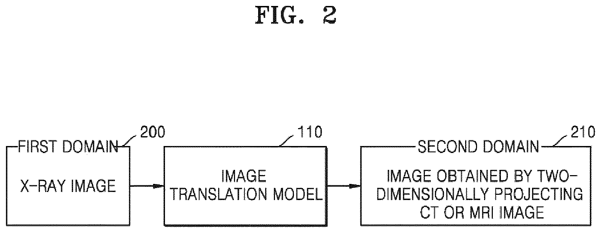

The image translation model 110 is a model implemented with an artificial intelligence network that converts a texture such as brightness while maintaining a structure of the first medical image 120. For example, the image translation model 110 may be a model that outputs the second medical image 130 obtained by harmonizing a brightness value of a 3D medical image such as a CT image with the first medical image 120 captured by an x-ray device. The term "structure" used herein refers to a shape that may distinguish shapes in a medical image, and the term "texture" refers to a brightness value of pixels filling the inside of the shape. For example, the image translation model 110 may output the second medical image 130 in which a lung shape in the first medical image 120 is maintained and a brightness value of the lung is reconstructed. The image translation model 110 will be described with reference to FIG. 2.

FIG. 2 is a diagram illustrating an example of an image translation model, according to an embodiment.

Referring to FIG. 2, the image translation model 110 is a model for translating an image of a first domain 200 into an image of a second domain 210. The image translation model 110 is a model for maintaining a structure shown in a medical image of the first domain 200 and reconstructing a texture such as a brightness value into a texture such as a brightness value of the second domain 210. In an embodiment, the first domain 200 may be a domain including a medical image captured by an x-ray device, and the second domain 210 may be a domain including an image obtained by two-dimensionally projecting a 3D medical image such as a CT or MRI image.

A 3D medical image may include a plurality of cross-sectional images, and a 2D projection image may be created by projecting the plurality of cross-sectional images onto a 2D plane. For example, a 2D projection image may be created by two-dimensionally projecting a 3D medical image of the chest.

A 3D medical image captured by a CT or MRI device includes voxels having a brightness value expressed in Hounsfield unit (HU), and an x-ray image includes pixels having a general brightness value. Accordingly, a brightness value of a 2D image captured by an x-ray device and a brightness value of an image obtained by projecting an image captured by a CT or MRI device onto a 2D plane may be different from each other. Accordingly, a better result may be obtained when applying a medical image (i.e., a projection image) of the second domain 210, rather than a medical image of the first domain 200, to an artificial intelligence model trained based on projection images of the second domain 210. Also, a better result may be obtained when inputting a medical image of the first domain 200, rather than a medical image (i.e., a projection image) of the second domain 210, to an artificial intelligence model trained based on x-ray images of the first domain 200.

Accordingly, the first domain 200 and the second domain 210 of the image translation model 110 may be pre-defined in various ways, and are not limited to the present embodiment. However, for convenience of explanation, it is assumed that the first domain 200 is a domain of an x-ray image, and the second domain 210 is a domain of an image obtained by two-dimensionally projecting a 3D medical image.

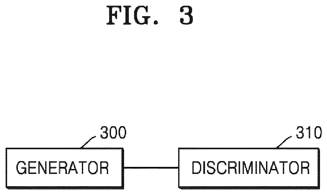

FIG. 3 is a diagram illustrating an example of a method of training an image translation model, according to an embodiment.

Referring to FIG. 3, the medical image translation apparatus 100 may train the image translation model 110 by using a generative adversarial network (GAN). The image translation model 110 corresponds to a generator 300 of the GAN. When the GAN is used, a medical image of a first domain and a medical image of a second domain which are unpaired may be used as training data. In another embodiment, when there are a pair of medical images between a first domain and a second domain, the image translation model 110 may be trained by using various supervised learning methods.

When the generator 300 receives a first medical image of a first domain, the generator 300 generates and outputs a second medical image of a second domain. A discriminator 310 determines whether the second medical image output from the generator 300 corresponds to a medical image of the second domain. Hereinafter, the medical image of the second domain used in a training process is referred to as a reference image. The generator 300 and the discriminator 310 of the GAN are trained through competition with each other. The generator 300 is trained about a feature of a texture such as a brightness value of the reference image through the training process. After completion of the training process, when the generator 300 receives the first medical image, the generator 300 generates the second medical image reconstructed by reflecting the feature such as the brightness value of the reference image in the first medical image. The training process of the generator 300 and the discriminator 310 of the GAN is already widely known, and thus, an additional description thereof will be omitted. When the training of the generator through the GAN is completed, the generator 300 may be used as the image translation model 110 of the present embodiment.

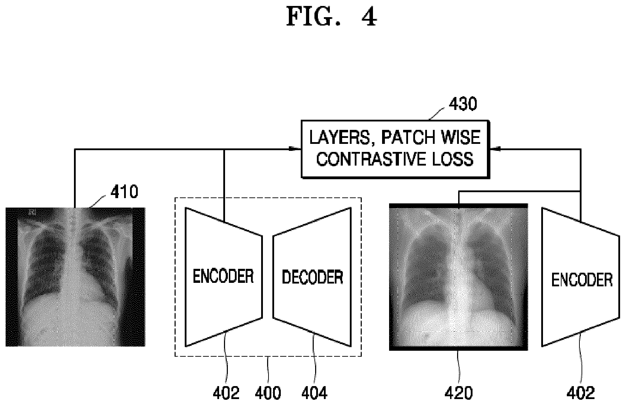

FIG. 4 is a diagram illustrating another example of a method of training an image translation model, according to an embodiment.

Referring to FIG. 4, an image translation model 400 includes an encoder 402 that extracts a feature from a first medical image 410 and a decoder 404 that generates a second medical image based on the feature extracted by the encoder 402. The encoder 402 and the decoder 404 each implemented with an artificial intelligence network are already widely known, and thus, an additional description thereof will be omitted. Various types of conventional encoders 402 and decoders 404 may be applied to the present embodiment.

An image translation model 400 including the encoder 402 and the decoder 404 may be trained as a generator (e.g., 300 of FIG. 3) of a GAN. That is, the image translation model 400 of the present embodiment may be replaced with the generator 300 of the GAN and may be trained by using the method of FIG. 3. In this case, in order to improve the performance of the image translation model, an additional loss function may be considered in addition to a loss function of the existing GAN.

A loss function 430 of the present embodiment includes a patch-wise contrastive loss with respect to a feature map of each layer of the encoder. A loss function may be obtained by summing values obtained by repeatedly performing, on layers of the encoder, a process of obtaining a patch-wise contrastive loss with respect to a feature map of a k^{th} layer of the encoder 402 appearing when the first medical image 410 is input to the encoder 402 and a feature map of a k^{th} layer of the encoder 402 appearing when a second medical image 420 is input to the encoder 402. The encoder 402 to which the second medical image 420 is input is the same as the encoder 402 to which the first medical image is input. In an embodiment, a patch-wise contrastive loss of each layer of the encoder 402 may be defined in contrastive unpaired translation (CUT). Also, the loss function 430 may be obtained by summing a patch-wise contrastive loss and a contrastive loss of each layer of the encoder for the first medical image 410 and the second medical image 420.

The medical image translation apparatus 100 may train the image translation model 400 by considering the loss function of FIG. 4 in the training process of the GAN of FIG. 3. The image translation model 400 may include the encoder 402 and the decoder 404 which have been trained.

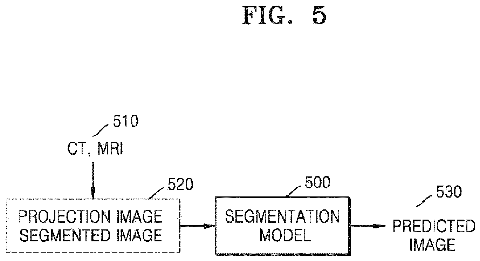

FIG. 5 is a diagram illustrating an example of a segmentation model, according to an embodiment.

Referring to FIG. 5, a segmentation model 500 is a model implemented with an artificial intelligence network that outputs an image 530 obtained by segmenting a human tissue or a human organ from a medical image. For example, the segmentation model 500 may be an artificial intelligence model that segments a lung tissue from a 2D medical image.

The segmentation model 500 is generated through a training process. For example, the segmentation model 500 may be trained by using a supervised learning method using training data including a 2D medical image and an image of a human tissue or a human organ as a pair. That is, the segmentation model 500 may be trained by using training data in which a 2D medical image is labeled with a segmented image of a human tissue or a human organ. Training is performed by comparing a predicted image 530 output from the segmentation model with the segmented image that is ground truth of the training data.

The segmented image of the human tissue or the human organ for labeling the training data may be manually segmented by a user from the 2D medical image. However, it may take a lot of time to manually generate the training data, and there may be a difference in the accuracy of the ground truth according to an operator's skill level.

Accordingly, the present embodiment proposes a method of generating training data based on a 3D medical image 510 such as a CT or MRI image. First, a human tissue or a human organ is segmented from the 3D medical image 510. For example, a 3D lung region may be segmented from the 3D medical image 510 by applying any of various conventional segmentation algorithms. Next, a projection image and a segmented image are respectively generated by two-dimensionally projecting the 3D medical image 510 and the 3D segmented region such as the human organ. A 2D image in which various tissues overlap each other like an x-ray image may be obtained by projecting a brightness value of each voxel of the 3D medical image onto a 2D plane. The segmentation model 500 may be trained by using, as training data 520, a pair of the segmented image obtained by two-dimensionally projecting the human tissue or the human organ (e.g., the lung region) segmented from the 3D medical image and the projection image obtained by two-dimensionally projecting the 3D medical image.

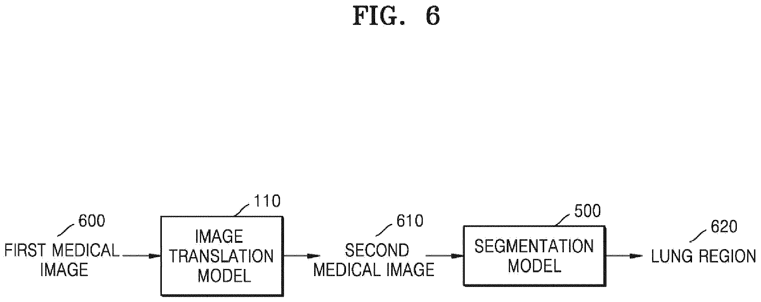

FIG. 6 is a diagram illustrating an example of a method of generating a medical image suitable for a segmentation model by using an image translation model, according to an embodiment.

Referring to FIG. 6, the segmentation model 500 is a model trained by using training data based on a 3D medical image described with reference to FIG. 5. The image translation model 110 is a model for translating an x-ray image of a first domain into a projection image of a second domain as described with reference to FIG. 2.

When the image translation model 110 receives a first medical image 600, the image translation model 110 translates the first medial image 600 into a second medical image 610. That is, a texture such as a brightness value is reconstructed by reflecting a feature of the projection image of the second domain 210 of FIG. 2 while a structure of the first medical image 600 is maintained. The segmentation model 500 receives the second medical image 610 and outputs an image obtained by segmenting a human tissue or a human organ (e.g., a lung region 620) from the second medical image 610.

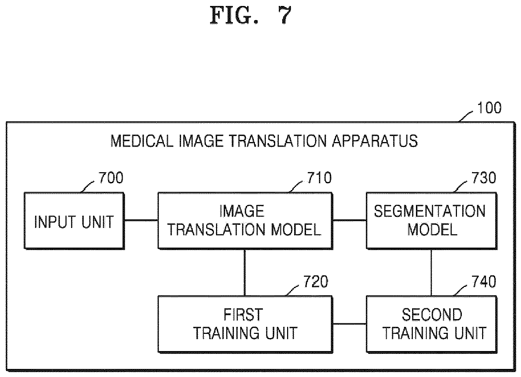

FIG. 7 is a diagram illustrating a configuration of an example of a medical image translation apparatus, according to an embodiment.

Referring to FIG. 7, the medical image translation apparatus 100 includes an input unit 700, an image translation model 710, a first training unit 720, a segmentation model 730, and a second training unit 740. In an embodiment, when the image translation model 710 and/or the segmentation model 730 has already been trained, the first training unit 720 and/or the second training unit 740 may be omitted. In another embodiment, the segmentation model 730 and the second training unit 740 may be omitted. Some of other elements may be omitted or other elements may be added. However, the following will be described assuming that all of the elements of FIG. 7 are included. Also, the medical image translation apparatus 100 may be implemented as a computing device including a memory, a processor, and an input/output device. In this case, each element may be implemented as software, may be loaded into the memory, and then may be executed by the processor.

The input unit 700 receives a first medical image. In an embodiment, the first medical image may be a 2D medical image captured by an x-ray device.

The image translation model 710 is a model for translating the first medical image into a second medical image. For example, the image translation model 710 is a model implemented with an artificial intelligence network that reflects and outputs a feature of a brightness value of each of pixels of a reference image used in a training process in a 2D medical image. An example of the image translation model 710 is illustrated in FIG. 2.

The segmentation model 730 is a model for segmenting a human tissue or a human organ from the second medical image reconstructed through the image translation model. The segmentation model 730 is a model implemented with an artificial intelligence network trained based on a projection image generated by two-dimensionally projecting a 3D medical image. An example of the segmentation model 730 is illustrated in FIG. 5.

The first training unit 720 trains the image translation model by using a GAN. In another embodiment, the first training unit 720 may use a loss function representing a contrastive loss of CUT in a training process of the GAN. An example of a method of training the image translation model 710 is illustrated in FIGS. 3 and 4.

The second training unit 740 trains the segmentation model 730 based on the projection image obtained by two-dimensionally projecting the 3D medical image. An example of a method of training the segmentation model 730 is illustrated in FIG. 5.

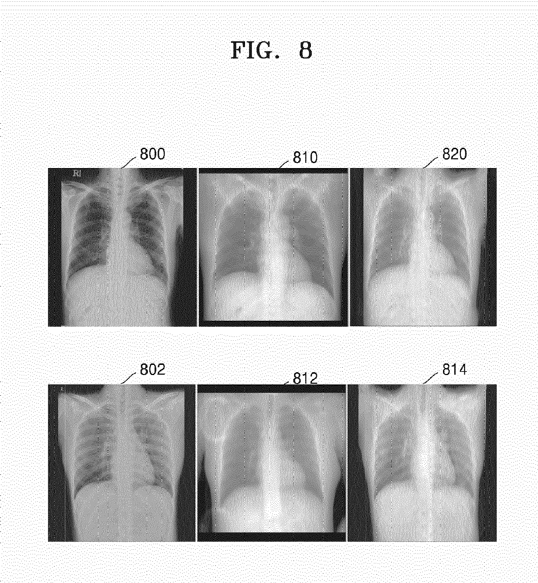

FIG. 8 is a view illustrating an example of a translation result of an image translation model, according to an embodiment.

Referring to FIG. 8, the image translation model 110 is a model trained about a feature of a texture such as a brightness value of each of projection images (i.e., reference images) 810 and 812 obtained by two-dimensionally projecting a 3D medical image. Accordingly, when the image translation model 110 receives first medical images 800 and 802, the image translation model 110 generates second medical images 820 and 814 reconstructed by reflecting the feature of the brightness value of each of the reference images 810 and 812 while maintaining a structure of each of the first medical images 800 and 802. That is, the first medical images 800 and 802 captured by an x-ray device are translated to look like projection images obtained by two-dimensionally projecting a 3D image through the image translation model 110.

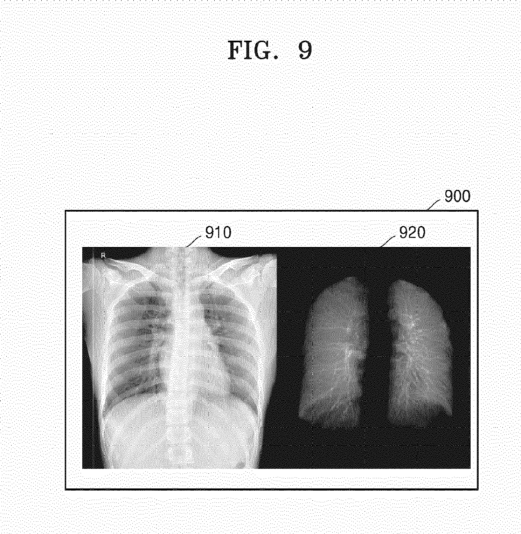

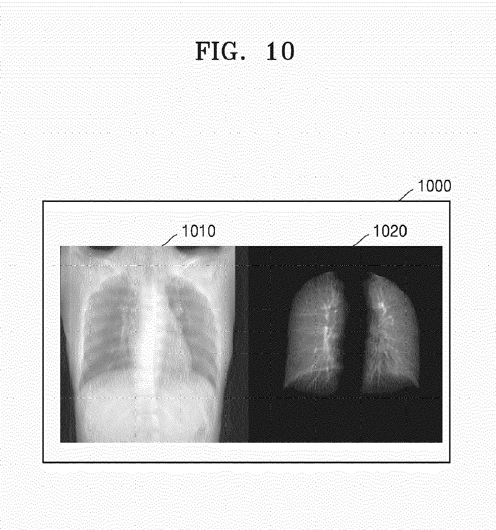

FIGS. 9 and 10 are views illustrating an example of a segmentation result of a segmentation model, according to an embodiment.

Referring to FIGS. 9 and 10, results 900 and 1000 obtained by segmenting a lung region from a medical image through the segmentation model 500 are illustrated. FIG. 9 is a view illustrating a lung region 920 segmented by inputting a first medical image 910 captured by an existing x-ray device to the segmentation model 500 trained by using the method of FIG. 5. FIG. 10 is a view illustrating a lung region 1020 segmented by inputting a second medical image 1010 translated from the first medical image 910 of FIG. 9 by the image translation model 110 of the present embodiment to the segmentation model 500. When the lung regions 920 and 1020 of FIGS. 9 and 10 are compared with each other, a boundary line of the lung and locations of blood vessels are more clearly seen in the image of FIG. 10.

The invention may also be implemented as computer-readable code on a computer-readable recording medium. The computer-readable recording medium includes any storage device that may store data which may be thereafter read by a computer system. Examples of the computer-readable recording medium include a read-only memory (ROM), a random-access memory (RAM), a compact disk (CD)-ROM, a magnetic tape, a floppy disk, and an optical data storage device. The computer-readable recording medium may also be distributed over network-coupled computer systems so that the computer-readable code is stored and executed in a distributive manner.

According to an embodiment, a medical image may be translated to be suitable for an artificial intelligence model. For example, when there is an artificial intelligence model trained by using, as training data, an image obtained by two-dimensionally projecting a 3D medical image captured by a CT device or an MRI device, a medical image captured by an x-ray image may be translated into a medical image suitable for input to the artificial intelligence model.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by one of ordinary skill in the art that various changes in form and details may be made therein without departing from the spirit and scope of the invention as defined by the following claims.

## Claims

1. Medical image translation method comprising:
receiving a first medical image; and
obtaining a second medical image by translating the first medical image through an image translation model,
wherein the first medical image is a two-dimensional (2D) medical image, and
the second medical image is a 2D medical image obtained by reconstructing a brightness value of each of pixels of the first medical image while maintaining a structure of the first medical image,
wherein the image translation model is a model implemented with an artificial intelligence network that reflects and outputs a feature of a brightness value of each of pixels of a reference image trained in a training process in a 2D medical image.

2. Medical image translation method of claim 1, wherein the reference image is an image generated by projecting a computed tomography (CT) image or a magnetic resonance imaging (MRI) image onto a 2D plane.

3. Medical image translation method of claim 1 or 2, further comprising training the image translation model by using a generative adversarial network (GAN).

4. Medical image translation method of claim 1 or 2, further comprising training the image translation model by using a contrastive unpaired translation (CUT).

5. Medical image translation method of any of claims 1 to 4, further comprising segmenting a human tissue or a human organ by inputting the second medical image to a segmentation model for segmenting a human tissue or a human organ from a medical image, wherein the segmentation model is a model implemented with an artificial neural network trained based on a projection image generated by two-dimensionally projecting a three-dimensional (3D) medical image.

6. Medical image translation method of claim 5, wherein the segmentation model is a model trained by using training data in which the projection image is labeled with a human tissue or a human organ segmented from the 3D medical image.

7. Medical image translation method of claim 5 or 6, wherein the segmentation model is a model for segmenting a lung region.

8. Medical image translation apparatus comprising:
an input unit (700) configured to receive a first medical image (120, 410, 600, 800, 802); and
an image translation model (110, 400, 710) configured to translate the first medical image into a second medical image (130, 420, 610, 820, 814),
wherein the first medical image is a two-dimensional (2D) medical image, and
the second medical image is a 2D medical image obtained by translating a brightness value of each of pixels of the first medical image while maintaining a structure of the first medical image,
wherein the image translation model is a model implemented with an artificial intelligence network that reflects and outputs a feature of a brightness value of each of pixels of a reference image used in a training process in a 2D medical image.

9. Medical image translation apparatus of claim 8, further comprising a segmentation model configured to segment a human tissue or a human organ from the second medical image, wherein the segmentation model is a model implemented with an artificial intelligence network trained based on a projection image generated by two-dimensionally projecting a three-dimensional (3D) medical image.

10. Medical image translation apparatus of claim 8, further comprising a training unit configured to train the image translation model by using a generative adversarial network (GAN) or contrastive unpaired translation (CUT).

11. Computer-readable recording medium having recorded thereon a computer program for performing the medical image translation method of any of claims 1 to 7.
